# EUROPÄISCHE PATENTSCHRIFT

(11) **EP 1 196 606 B1**
(45) Veröffentlichungstag und Bekanntmachung des Hinweises auf die Patenterteilung: **17.11.2004**
(21) Anmeldenummer: 00942146.2
(22) Anmeldetag: 28.06.2000
(51) Int. Cl.: C12N 15/55, C12N 9/24, C12N 1/20, C12N 1/21, C12N 1/06, C07K 16/40

(54) **LYTISCHES ENZYM**
LYTIC ENZYME
ENZYME LYTIQUE

(30) Priorität: 28.06.1999 DE 19929485
(43) Veröffentlichungstag der Anmeldung: 17.04.2002
(73) Patentinhaber: Fraunhofer-Gesellschaft zur Förderung der angewandten Forschung e.V., 80686 München (DE)
(72) Erfinder: TRICK, Iris, Ruth, D-75305 Neuenbürg (DE); WEBER, Hans, D-71642 Ludwigsburg (DE); SASSE, Julia, D-70563 Stuttgart (DE)
(86) Internationale Anmeldenummer: PCT/EP2000/006000
(87) Internationale Veröffentlichungsnummer: WO 2001/000850

(56) Entgegenhaltungen:
- EP-A- 0 506 448
- EP-A- 0 577 326
- WO-A-99/29841
- NONAKA T. ET AL.: "Amino acid sequences of metalloendopeptidases specific for acyl-lysine bonds from Grifola frondosa and Pleurotus ostreatus fruiting bodies." J. BIOL. CHEM., Bd. 272, Nr. 48, 28. November 1997 (1997-11-28), Seiten 30032-30039, XP002150254
- EPSTEIN D.M. & WENSINK P.C.: 'The alpha-lytic protease gene of lysobacter enzymogenes' J. BIOL. CHEM. Bd. 263, Nr. 32, 1988, Seiten 16586 - 16590, XP002150289

### SEQUENZPROTOKOLL

<110> Fraunhofer-Ges. zur Förderung der angewandten Forschung e.V.
<120> Lytisches Enzym aus Lysobacter enzymogenes
<130> 23395
<140>
   <141>
<150> 19929485.2-41 (DE)
   <151> 1999-06-28
<160> 7
<170> PatentIn Ver. 2.1
<210> 1
   <211> 39
   <212> PRT
   <213> Lysobacter enzymogenes
<400> 1
   Ala Val Pro Asn Gly Val Asn Tyr Val Gly Xaa Thr Ser Ala Arg Thr
    1               5                  10                  15
   Ser Xaa Ala Gly Gln Ala Val Xaa Gln Ala Arg Xaa Tyr Xaa Glu Asn
               20                  25                  30
   Ala Lys Gly Tyr Leu Asn Gly
           35
<210> 2
   <211> 117
   <212> DNA
   <213> Lysobacter enzymogenes
<400> 2
   gcngtncana ayggngtnaa ytaygtnggn nnnacntcng cnmgnacntc nnnngcnggn 60
   cangcngtnn nncargcncg nnnntaynnn garaargcna arggntayyt naayggn    117
<210> 3
   <211> 18
   <212> PRT
   <213> Lysobacter enzymogenes
<400> 3
   Ala Asn Ile Val Gly Leu Ile Glu Tyr Ser Ile Asn Asn Ala Ser Leu
    1               5                  10                  15
   Xaa Ser
<210> 4
   <211> 48
   <212> DNA
   <213> Lysobacter enzymogenes
<400> 4
   gcnaanatng tnggnctnat ngantannnn atnaanaang cnnnnctn              48
<210> 5
   <211> 5
   <212> PRT
   <213> Lysobacter enzymogenes
<220>
   <221> PEPTIDE
   <222> (2)
   <223> Xaa ist Phe oder Asp
<400> 5
   Thr Xaa Trp Asn Ala
    1               5
<210> 6
   <211> 7
   <212> PRT
   <213> Lysobacter enzymogenes
<220>
   <221> PEPTIDE
   <222> (3)
   <223> Xaa ist Glu oder Cys
<400> 6
   Phe Ala Xaa Asn Thr Pro Phe
    1               5
<210> 7
   <211> 4
   <212> PRT
   <213> Lysobacter enzymogenes
<400> 7
   Tyr Thr Thr Trp
    1

## Beschreibung

Die vorliegende Erfindung betrifft ein Protein mit der Aktivität eines lytischen Enzyms, einen dieses Enzym bildenden Mikroorganismus, dieses Enzym codierende Nucleinsäuremoleküle, diese Nucleinsäuremoleküle enthaltende Vektoren, diese Vektoren enthaltende Wirtszellen, Antikörper gegen dieses lytische Enzym, Proteingemische, enthaltend dieses Enzym sowie Verfahren zur Herstellung des lytischen Enzyms und deren Anwendung.

Eine Reihe von Mikroorganismen weist die Fähigkeit auf, durch die Bildung lytischer Enzyme die Zellwände anderer Organismen aufzulösen. Myxobakterien oder gleitenden Bakterien, insbesondere Bakterien der Gattung Lysobacter, kommt hinsichtlich der Bildung lytischer Enzyme eine besonders große Bedeutung zu. Myxobakterien zeichnen sich durch ein breites Wirkungsspektrum gegen verschiedene Organismen aus. So bilden diese Bakterien hefelytische Enzyme ebenso wie lytische Enzyme gegen Gram-positive oder Gram-negative Bakterien. Ebenso konnte lytische Aktivität gegen Pilze, Algen oder Chitin beobachtet werden. Arten der Gattung Lysobacter sind nur teilweise befähigt, Enzyme gegen Gram-negative Bakterien zu produzieren. Sie bilden jedoch Enzyme, die Chitin spalten können, so dass Hefen und Pilze, die eine Zellwand aus Chitin besitzen, aufgelöst werden. Mitsutani, J. of National Fisheries University 45(4) (1997), 165-257, konnte mittels Lysobacter LB1 gute Abbauergebnisse von Algenblüten von Cyanobakterien in Süßwasserseen zeigen.

Aus Lysobacter enzymogenes 495 sind bisher drei verschiedene Proteasen bekannt. Eine α-lytische Protease, eine β-lytische Metallo-Endopeptidase und eine Lys-C-Endoproteinase. Die α-lytische Protease, beschrieben in Epstein und Wensink, J. of Biol. Chem. 263 (32), 1988, 16586-16590, ist eine Serin-Protease, deren Kennzeichen ein besonders reaktiver Serin-Rest im katalytischen Zentrum ist. Die β-lytische Metallo-Endopeptidase, beschrieben in Damaglou et al., Proceedings of the Candian Federation Biological Society 15(2), 1974, ist eine N-acetylmuramoyl-L-Alanin-Amidase, die die Bindung zwischen Polysaccharid und Peptid hydrolysiert. Zusammen mit der α-lytischen Protease werden durch sie nicht nur Gram-positive, sondern auch Gram-negative Bakterien lysiert (Li et al., J. Bacteriol. 172 (11) (1990), 6506-6511. Ebenfalls eine Serin-Protease ist die Lys-C-Endopeptidase, die die Ester- und Amid-Bindungen an der Carboxyseite von Lysin spaltet (Reichenbach in "Biotechnologie", VCH-Verlag, Weinheim (1988), Kapitel 20, 674-696). Aus L. enzymogenes AL-1 sind zwei verschiedene Proteasen bekannt, nämlich die AL-1-Protease 1, die die Pentaglycinbrücken in der Wand von S. aureus und das Acetylmuramyl L-Alanin von M. lysodeikticus und S. aureus-Zellwänden spaltet (Tipper et al., Biochemistry 6 (1967), 906-920). Die AL-1 Protease 2 spaltet zwar keine Zellwände, dafür aber verschiedene Proteine mit hoher Spezifität zur Aminoseite von Lysin (Hedges und Wolfe, J. Bacteriol. 120, 844-853 (1974), Extracellular enzyme from Myxobacter AL1 that exhibits both β-1,4,-glucanase and chitosanase activities).

Der enzymatische Aufschluss mikrobieller Zellen unterschiedlicher Spezies oder von isolierten Makromolekülen wie Chitin oder Cellulose kann also durch eine Reihe unterschiedlicher Präparate, insbesondere Enzyme, erreicht werden. Die Anforderungen an die Enzyme orientieren sich dabei stark an dem speziellen Reaktionsschritt, der katalysiert werden soll und an den prozessabhängigen weiteren Umgebungsbedingungen. Daher ergibt sich für lytische Enzyme jeweils nur ein relativ begrenztes Einsatzgebiet. Es besteht daher ein ständiger Bedarf an der Bereitstellung weiterer lytischer Enzyme.

Das der vorliegenden Erfindung zugrundeliegende technische Problem besteht also darin, ein weiteres lytisches Enzym bereitzustellen, das in der Lage ist, Zellen, insbesondere mikrobielle Zellen, Makromoleküle oder sonstige biologische Materialien zu spalten.

Die Erfindung löst dieses technische Problem durch die Bereitstellung eines isolierten und im wesentlichen frei von anderen Substanzen, insbesondere Proteinen oder Peptiden, vorliegenden Proteins mit der Aktivität eines lytischen Enzyms, welches zumindest die in SEQ ID Nr. 1, 5, 6 und/oder 7 dargestellte Aminosäuresequenz umfasst. Die Erfindung löst dieses Problem auch durch die Bereitstellung eines vorgenannten Proteins mit der Aktivität eines lytischen Enzyms, wobei das Protein ein Molekulargewicht, bestimmt mittels HPLC und/oder MALDI TOF (Matrix assisted laser desorption ionization, time of flight, Gerät: Hewlett Packard G2025A LD-TOF System), von 15000 bis 20000 Da, ein Temperaturoptimum von 37°C und ein pH-Optimum von 9,0 bis 10,0 aufweist. Die Erfindung betrifft selbstverständlich auch mutante Varianten oder Derivate dieser Proteine, die sich, bei Erhöhung oder im wesentlichen bei Beibehaltung ihrer natürlichen enzymatischen Aktivität, durch chemische Modifikation der Aminosäuren und/oder eine geänderte Aminosäuresequenz auszeichnen.

Im Zusammenhang mit der vorliegenden Erfindung wird unter einem Protein mit der Aktivität eines lytischen Enzyms ein Protein, ein Peptid oder ein Peptidfragment verstanden, das in der Lage ist, biologische Materialien wie Zellen, insbesondere mikrobielle Zellen, bevorzugt Gram-positive und/oder Gram-negative Zellen sowie gegebenenfalls Actinomyceten, Nematoden, Pilze, Hefen, Algen, Cellulose, Chitin und/oder Murein enzymatisch zu spalten, insbesondere die Zellwand oder diese aufbauende Substanzen im Zellwandgerüst oder isoliert zu spalten, das heißt in kleinere molekulare Einheiten zu zerlegen. Neben einem Protein mit der in der SEQ ID Nr. 1, 5, 6 und/oder 7 gezeigten Aminosäuresequenz umfasst die Erfindung selbstverständlich auch lytische Enzyme mit Abweichungen von dieser Sequenz, wobei diese Abweichungen Deletionen, Inversionen oder Insertionen von Aminosäuren, auch durch selten vorkommende oder unnatürliche, also synthetische Aminosäuren, oder ähnliches darstellen können. Die Erfindung betrifft auch Derivate der vorgenannten Proteine, die sich durch chemische Modifikationen, zum Beispiel Alkylierung einer oder mehrerer Aminosäuren auszeichnen. Das erfindungsgemäße Protein mit der Aktivität eines lytischen Enzyms kann sowohl natürlicher als auch synthetischer Herkunft sein und gegebenenfalls als Fusionsprotein oder Fusionspeptid mit Peptiden oder Proteinen anderer enzymatischer Aktivität vorliegen. Das erfindungsgemäße Protein kann auch als Pro-, Prä- oder Präproprotein vorliegen, das heißt zusätzliche Aminosäuresequenzen können vorgesehen sein, die für Transport-, Lokalisierungs- und/oder Konformationszwecke notwendig sind und gegebenenfalls im reifen Protein nicht mehr vorhanden sind. Die verschiedenen Varianten und Derivate der erfindungsgemäßen Proteine weisen bestimmte gemeinsame Charakteristika auf, wie die Enzymaktivität, das Molekulargewicht, immunologische Reaktivität und/oder deren Konformation oder physikalische Eigenschaften wie das Laufverhalten in Gelelektrophoresen, chromatographisches Verhalten, Sedimentationskoeffizienten, spektroskopische Eigenschaften, Stabilität, pH-Optimum, Temperaturoptimum oder Löslichkeit.

Das erfindungsgemäße Protein beziehungsweise Enzym zeichnet sich durch eine hohe lytische Aktivität gegenüber Gram-negativen und eine gute lytische Aktivität gegenüber Gram-positiven Bakterien aus. Das erfindungsgemäße Enzym ist über einen pH-Wert-Bereich von pH 6 bis pH 12 stabil, wobei in bevorzugter Ausführungsform eine Temperatur-Stabilität über fünf Tage bei Temperaturen bis 37°C gegeben ist. Das erfindungsgemäße Enzym kann vorteilhafterweise zum Aufschluss von Zellen eingesetzt werden. Selbstverständlich kann auch eine enzymatische Vorbehandlung von Klärschlamm mittels des erfindungsgemäßen Enzyms vorgesehen werden, um die anaerobe Umsetzung beispielsweise in einem Faulturm zu erhöhen. Erfindungsgemäß konnte gezeigt werden, dass in Belebtschlamm vorkommende Organismen durch das erfindungsgemäße Enzym lysiert werden konnten.

In einer weiteren bevorzugten Ausführungsform betrifft die vorliegende Erfindung einen Mikroorganismus, der das erfindungsgemäße lytische Enzym bildet, insbesondere in das extrazelluläre Medium sezerniert. Der erfindungsgemäße Mikroorganismus ist ein Gram-negatives, Oxidase-positives, stäbchenförmiges Bakterium mit strikt aerober Lebensweise. Die Enzymaktivität des erfindungsgemäßen Proteins lag überraschenderweise über der der lytischen Enzyme der Vergleichsstämme L. enzymogenes 495 und L. enzymogenes AL1. Der erfindungsgemäße Mikroorganismus kann Cellobiose und Chitin verwerten, nicht jedoch Mannose, Xylose, Cellulose oder Carboxymethylcellulose. Der erfindungsgemäße Mikroorganismus kann bei einer Natriumchloridkonzentration von 7 % oder mehr nicht wachsen. Mittels des OF-Tests (Oxidations-Fermentations-Test nach Hugh und Leifson; Süßmuth et al., 1987, "Biochemischmikrobiologisches Praktikum") charakterisiert sich der erfindungsgemäße Organismus als Oxidativpositiv und Fermentativ-negativ, wobei der Voges-Proskauer-Test zum Glucosestoffwechsel negativ verläuft. Der erfindungsgemäße Organismus bildet weder Sulfid noch Indol. Der erfindungsgemäße Organismus weist in Kolonieform gleitende Ausläufer am Kolonierand auf, wobei auch ältere Kolonien weder Furchen aufweisen noch gelappt erscheinen. Nach zwei Wochen Inkubation auf einer Nährmediumplatte setzt Autolyse ein. Eine Gleitbewegung des Mikroorganismus in Richtung nährstoffreicheren Mediums kann festgestellt werden. Der erfindungsgemäße Mikroorganismus weist eine schlanke lange Zellform mit einer Länge von bis zu 4 µm auf, wobei kein Hinweis auf eine Begeißelung festgestellt werden konnte. In einer besonders bevorzugten Ausführungsform der Erfindung weist das erfindungsgemäße Bakterium sehr gute lytische Aktivität gegenüber Gram-positiven Bakterien wie Micrococcus luteus und Bacillus subtilis, gute lytische Aktivität gegenüber der Hefe Saccharomyces cerevisiae und lytische Aktivität gegenüber den Gram-negativen Bakterien Escherichia coli und Pseudomonas acidovorans auf. Der erfindungsgemäße Mikroorganismus sowie damit durchgeführte Abbau- beziehungsweise Aufschlussverfahren können in bevorzugter Ausführung in Belebtschlamm oder ausgefaultem Schlamm eingesetzt werden.

In einer vorteilhaften, besonders bevorzugten Ausgestaltung der Erfindung handelt es sich bei dem vorgenannten erfindungsgemäßen Organismus um eine Art aus der Gattung Lysobacter enzymogenes. In einer weiteren bevorzugten Ausführungsform handelt es sich bei dem erfindungsgemäßen Mikroorganismus insbesondere um eine bei der DSMZ in Braunschweig unter der Zugangs-Nr. DSM 12887 hinterlegte Art oder ein davon abgeleitetes Bakterium.

Die vorliegende Erfindung betrifft auch ein Verfahren zur Herstellung des erfindungsgemäßen Proteins mit der Aktivität eines lytischen Enzyms, wobei ein erfindungsgemäßer Mikroorganismus der vorgenannten Art kultiviert wird, und zwar unter Bedingungen, die die Bildung des erfindungsgemäßen Enzyms und vorzugsweise dessen Sezernierung ins extracelluläre Kulturmedium erlauben und wobei das so hergestellte Enzym aus dem Kulturmedium isoliert wird. Das erfindungsgemäße Verfahren wird vorteilhafterweise in besonders bevorzugter Ausführungsform bei einem pH-Wert von 5,75 bis 10,0, insbesondere 7,5 bis 8,0, besonders bevorzugt 7,7, durchgeführt. In einer weiteren bevorzugten Ausführungsform wird das erfindungsgemäße Verfahren bei einer Temperatur von 25 bis 30°C, insbesondere 30°C, durchgeführt.

Unter diesen Bedingungen werden auch zwei weitere lytische Enzyme von dem erfindungsgemäßen Organismus gebildet, nämlich eine aus Damaglou et al., a.a.O. bekannte β-lytische Metallo-Endopeptidase von L. enzymogenes mit einem Molekulargewicht von 19100 Da, bestimmt durch Gelchromatographie. Das weitere gebildete Enzym ist eine bisher noch nicht bekannte α-lytische Protease, die eine 83%ige Aminosäuresequenzidentität zu der bekannten α-lytischen Protease (vergleiche Epstein und Wensink, a.a.O.) von L. enzymogenes 495 aufweist und deren Aminosäuresequenz teilweise in SEQ ID Nr. 3 dargestellt ist. Die Erfindung betrifft auch diese α-lytische Protease sowie deren mutante Varianten und Abweichungen sowie die vorgenannte Protease codierende Nucleinsäuresequenzen, diese enthaltende Vektoren und damit transformierte Zellen. Die vorgenannten drei Enzyme lassen sich durch übliche Ionenaustausch-Chromatographie und Gelchromatographie voneinander trennen.

Die Erfindung betrifft daher auch das durch das erfindungsgemäße Verfahren gebildete Enzym mit der Sequenz aus SEQ ID Nr. 1, 5, 6 und/oder 7 sowie Gemische dieses Enzyms mit mindestens einer, vorzugsweise beiden der vorgenannten α- und β-lytischen Proteasen. Selbstverständlich sind auch Gemische mindestens zweier der vorgenannten drei Enzyme erfindungsgemäß erfasst, bei denen mindestens eine der beteiligten Enzyme eine mutante Variante oder ein Derivat des jeweiligen Wildtyp-Proteins darstellt.

In einer weiteren bevorzugten Ausführungsform betrifft die Erfindung auch die gentechnische Herstellung der erfindungsgemäßen Enzyme mit den Aminosäuresequenzen aus SEQ ID Nr. 1, 3, 5, 6 und/oder 7 oder der Gemische mit mindestens einer der jeweils beiden anderen Enzyme und die dabei eingesetzten Mittel wie Nucleinsäuremoleküle, diese enthaltende Vektoren, diese enthaltende Wirtszellen sowie Verfahren zur Kultivierung dieser Wirtszellen und zur Gewinnung von Enzymen aus den Wirtszellen.

Demgemäß betrifft die Erfindung in einer bevorzugten Ausführungsform mindestens ein isoliertes Nucleinsäuremolekül, codierend mindestens ein Protein mit der Aktivität eines lytischen Enzyms, insbesondere mindestens eines der vorgenannten Enzyme, ausgewählt aus der Gruppe bestehend aus
a) Nucleinsäuremolekülen, die ein Protein codieren, umfassend eine oder mehrere Aminosäuresequenzen, ausgewählt aus der Gruppe bestehend aus SEQ ID Nr. 1, 3, 5, 6 und 7 oder deren komplementäre Sequenzen;
b) Nucleinsäuremolekülen, die die unter SEQ ID Nr. 2 oder 4 angegebene Nucleinsäuresequenz umfassen oder deren komplementäre Sequenz,
c) Nucleinsäuremolekülen, die in dem unter der Nr. DSM 12887 bei der DSMZ hinterlegten Mikroorganismus enthalten sind und mit der Sequenz aus a) oder b) hybridisieren,
d) Nucleinsäuremolekülen, die mit einer der unter a), b) oder c) genannten Nucleinsäuremoleküle hybridisieren, wobei in bevorzugter Weise das von diesen Nucleinsäuremolekülen codierte lytische Enzym eine Aminosäuresequenz aufweist, die zu mindestens 90% identisch zu der in SEQ ID Nr. 1, 3, 5, 6 oder 7 angegebenen Aminosäuresequenz ist, und
e) Nucleinsäuremolekülen, die aufgrund der Degeneration des genetischen Codes von der Sequenz unter a), b), c) oder d) abweichen.

In einer besonders bevorzugten Ausführungsform der Erfindung ist das Nucleinsäuremolekül ein DNA-Molekül oder ein RNA-Molekül. Das erfindungsgemäße Nucleinsäuremolekül kann natürlicher oder synthetischer Herkunft sein und auch ungewöhnliche Nucleotide aufweisen. Das erfindungsgemäße Nucleinsäuremolekül ist in vorteilhafter Weise ein aus dem vorgenannten Mikroorganismus, insbesondere hinterlegt bei der DSMZ unter der Nr. DSM 12887, stammendes Nukleinsäuremolekül. Mittels gängiger molekularbiologischer Techniken, wie sie in Sambrook et al., 1989, Molecular Cloning, A Laboratory Manual, 2. Auflage. Cold Spring Harbor, Laboratory Press, NJ, beschrieben sind, ist es möglich, verschiedenartige Mutationen in den erfindungsgemäßen Nucleinsäuremolekülen einzuführen, wodurch es zur Synthese von Proteinen mit eventuell veränderten biologischen Eigenschaften kommt. Die vorliegende Erfindung betrifft demgemäß auch Abwandlungen, Variationen, Derivate oder Modifikationen der vorgenannten Nucleinsäuresequenz, wobei diese Abwandlung in Additionen, Deletionen, Inversionen oder Austauschen von einzelnen oder mehreren Nucleotiden bestehen kann. Selbstverständlich sind auch Abwandlungen oder Derivatisierungen der Nucleotide selbst erfindungsgemäß erfasst. Die Erfindung betrifft auch chimäre Konstrukte aus den erfindungsgemäßen Nucleinsäuresequenzen sowie weiteren Nucleinsäuresequenzen, die, vorzugsweise im Leseraster, an die erfindungsgemäßen codierenden Sequenzen fusioniert werden und so ein Fusionsprotein oder Fusionspeptid codieren. Erfindungsgemäß sind auch Deletionsmutanten erfasst, bei denen durch fortschreitende Deletionen vom 5'- oder vom 3'-Ende der codierenden Nucleinsäuresequenz Nucleinsäuremoleküle erzeugt werden, die zur Synthese entsprechend verkürzter Proteine führen. Die erfindungsgemäßen Nucleinsäuremoleküle können auch zu Nucleinsäuremolekülen fusioniert werden, deren codierte Peptide zu einer bestimmten Konformation, Lokalisierung und/oder zu einem gezielten Transport in Kompartimente oder extracelluläre Bereiche des Wirtsorganismus führen können.

Im Zusammenhang mit der vorliegenden Erfindung wird unter dem Begriff Hybridisierung eine Hybridisierung unter konventionellen Hybridisierungsbedingungen verstanden, vorzugsweise unter stringenten Bedingungen, wie sie in Sambrook et al., a.a.O. beschrieben sind.

Die erfindungsgemäßen Nucleinsäuremoleküle können zur Identifizierung, Clonierung und Isolation von lytische Enzyme codierenden Nucleinsäuremolekülen in den verschiedensten Organismen dienen. Als derartige Hybridisierungssonde können zum Beispiel Nucleinsäuremoleküle verwendet werden, die exakt die oder im wesentlichen die unter SEQ ID Nr. 2 oder 4 angegebene Nucleotidsequenz oder Teile dieser Sequenzen aufweisen. Bei den als Hybridisierungssonde verwendeten Fragmenten kann es sich auch um synthetische Fragmente handeln, die mit Hilfe üblicher Synthesetechniken hergestellt wurden und deren Sequenz im wesentlichen mit der eines erfindungsgemäßen Nucleinsäuremoleküls übereinstimmt. Die Erfindung umfasst selbstverständlich auch Oligonucleotide, beispielsweise mit einer Länge von mindestens 10, vorzugsweise von mindestens 15 und besonders bevorzugt von mindestens 40 Nucleotiden, die mit den erfindungsgemäßen Nucleinsäuremolekülen hybridisieren und beispielsweise als Primer für PCR-Reaktionen oder als Clonierungssonden verwendet werden können.

Die mit den erfindungsgemäßen Nucleinsäuremolekülen hybridisierenden Moleküle umfassen also auch Fragmente, Derivate und auch allelische Varianten der oben beschriebenen Nucleinsäuremoleküle, die ein erfindungsgemäßes Protein codieren. Unter Fragmenten werden dabei erfindungsgemäß Teile der Nucleinsäuremoleküle verstanden, die lang genug sind, um eines der beschriebenen Proteine zu codieren. Der Ausdruck allelische Variante bedeutet in diesem Zusammenhang, dass die Sequenzen dieser Moleküle sich von den Sequenzen der oben beschriebenen Nucleinsäuremoleküle an einer oder mehreren Positionen unterscheiden, gleichwohl aber noch einen hohen Grad an Homologie zu diesen Sequenzen aufweisen. Erfindungsgemäß wird unter dem Begriff hohe Homologie eine Sequenzidentität von mindestens 40%, insbesondere eine Identität von mindestens 60%, besonders bevorzugt über 80% und ganz besonders bevorzugt über 90% verstanden. Selbstverständlich ist es möglich, durch gentechnische Methoden die Aktivität oder das Aktivitätsprofil, beispielsweise die Substratspezifizität, des erfindungsgemäßen Proteins zu ändern, insbesondere zu verbessern beziehungsweise zu erhöhen.

Die erfindungsgemäßen Proteine mit der Aktivität eines lytischen Enzyms weisen in bevorzugter Ausführungsform eine Sequenzidentität zu der in SEQ ID Nr. 1, 3, 5, 6 und/oder 7 angegebenen Aminosäuresequenz von mindestens 80%, vorzugsweise 85%, besonders bevorzugt über 90%, 95%, 97% und 99% auf Aminosäureebene auf.

Die Erfindung betrifft in einer weiteren bevorzugten Ausführungsform Vektoren, die die vorgenannten Nucleinsäuremoleküle enthalten. Im Zusammenhang mit der vorliegenden Erfindung werden unter Vektoren Plasmide, Viren, Bacteriophagen, Cosmide oder andere Vektoren verstanden. Die Erfindung sieht in einer weiteren bevorzugten Ausführungsform vor, dass die erfindungsgemäßen Nucleinsäuremoleküle im erfindungsgemäßen Vektor mit regulatorischen Elementen operativ so verbunden sind, dass die Transcription und Synthese einer translatierbaren RNA in pro- und/oder eucaryotischen Zellen möglich ist. Die erfindungsgemäßen Nucleinsäuremoleküle werden vorteilhafterweise in Transcriptionseinheit mit 5'-regulatorischen Nucleinsäuresequenzen verbunden, die auch als Promoter bezeichnet werden und eine Expression in einer Wirtszelle erlauben. Erfindungsgemäß kann in einer weiteren bevorzugten Ausführungsform vorgesehen sein, weitere regulatorische Elemente einzusetzen, die beispielsweise im 5'-Bereich oder/und im 3'-Bereich der codierenden erfindungsgemäßen Nucleinsäuremoleküle vorhanden sind, beispielsweise Enhancer oder Transcriptionsterminationssignale.

Die erfindungsgemäßen Expressionsvektoren erlauben die Herstellung erfindungsgemäßer Proteine in verschiedenen Wirten, beispielsweise Escherichia coli.

Selbstverständlich können die erfindungsgemäßen Vektoren auch eine oder weitere Funktionseinheiten aufweisen, zum Beispiel Sequenzen, die eine Stabilisierung des Vektors im Wirtsorganismus bewirken, ein bakterieller Replikationsursprung, Selektionsmarker oder ähnliche Sequenzen.

Es kann auch vorgesehen sein, in einem Vektor verschiedene Transcriptionseinheiten und/oder verschiedene Translationseinheiten anzuordnen. Zum Beispiel ist es erfindungsgemäß vorgesehen, die codierenden Bereiche für die erfindungsgemäßen Proteine mit den Sequenzen aus SEQ ID Nr. 1, 3, 5, 6 und/oder 7, gegebenenfalls zusammen mit der codierenden Sequenz für die β-lytische Metallo-Endopeptidase, in Transcriptionseinheit in einem Vektor anzuordnen, wobei diese zwei oder drei codierenden Sequenzen jeweils zusammen mit entsprechenden operativ verbundenen regulatorischen Elementen angeordnet sind.

In einer weiteren bevorzugten Ausführungsform der Erfindung betrifft diese Wirtszellen, die die erfindungsgemäßen Nucleinsäuremoleküle oder die erfindungsgemäßen Vektoren transient oder stabil enthalten.

Im Zusammenhang mit der vorliegenden Erfindung wird unter einer Wirtszelle ein Organismus verstanden, der die erfindungsgemäßen Nucleinsäuremoleküle oder Vektoren enthält und gegebenenfalls die von der erfindungsgemäßen Nucleinsäure codierten Proteine synthetisieren kann. Die Erfindung betrifft in vorteilhafter Weise procaryotische oder eucaryotische Wirtszellen, zum Beispiel Escherichia coli, Lysobacter enzymogenes oder Hefe. Die erfindungsgemäßen Wirtszellen sind bevorzugt dadurch ausgezeichnet, dass das eingeführte erfindungsgemäße Nucleinsäuremolekül entweder heterolog in bezug auf die transformierte Zelle ist, das heißt natürlicherweise nicht oder nicht in der Kombination mit den verwendeten regulatorischen Elementen in dieser Zelle vorkommt und/oder an einem anderen Ort im Genom und/oder in einer anderen Kopienzahl als natürlicherweise vorkommt.

Die Erfindung betrifft daher auch ein Verfahren zur Herstellung mindestens eines der vorgenannten Proteine mit der Aktivität eines lytischen Enzyms, wobei eine Wirtszelle der vorgenannten Art unter Bedingungen kultiviert wird, die eine Bildung des mindestens einen erfindungsgemäßen Proteins ermöglicht und das gebildete Protein oder Proteingemisch aus dem Kulturmedium isoliert wird. Die vorliegende Erfindung betrifft daher auch gemäß dieses Verfahrens gewonnene Proteine oder Proteingemische.

Die Transformation eines Wirtsorganismus mit dem erfindungsgemäßen Nucleinsäuremolekül kann mittels gängiger Methoden, wie sie beispielsweise in Sambrook et al., a.a.O. beschrieben sind, durchgeführt werden.

Die Erfindung betrifft auch monoclonale oder polyclonale Antikörper, die spezifisch mit einem der erfindungsgemäßen Proteine reagieren, insbesondere dieses erkennen und binden.

Schließlich betrifft die Erfindung auch Verfahren zum Aufschluss von biologischem Material, insbesondere Zellen oder Makromolekülen, wobei ein erfindungsgemäßes Bakterium und/oder ein erfindungsgemäßes Protein oder Proteingemisch der vorgenannten Art mit dem aufzuschließenden biologischen Material, insbesondere den Zellen, inkubiert und das biologische Material aufgeschlossen wird. Selbstverständlich ist es möglich, den beschriebenen Zellaufschluss mittels weiterer Verfahrensmaßnahmen zu unterstützen, beispielsweise durch Einsatz chemischer Agentien und/oder elektrischer Felder.

Die Erfindung betrifft auch ein Verfahren zum Abbau von Klärschlamm, wobei Bakterien und/oder Proteine oder Proteingemische der vorliegenden Erfindung dem Klärschlamm zugefügt und inkubiert werden. Eine derartige enzymatische Vorbehandlung von Klärschlamm verbessert die anaerobe Umsetzung beispielsweise in einem Faulturm, da Belebtschlammorganismen lysiert werden. Erfindungsgemäß kann auch vorgesehen werden, den erfindungsgemäßen Mikroorganismus in Verfahren zur Behandlung von mikrobiell kontamierten Oberflächen zum Beispiel Lebensmitteln oder Bausubstanzen einzusetzen mit dem Ziel, diese von unerwünschten Mikroorganismen zu befreien. Zudem können mit den erfindungsgemäßen Mikroorganismen Fermentationsrückstände umgewandelt, insbesondere abgebaut werden, zum Beispiel partikuläre Bestandteile in lösliche Stoffe. Schließlich kann der erfindungsgemäße Mikroorganismus in Zellaufschlussverfahren für zum Beispiel biochemische oder ähnliche Anwendungen eingesetzt werden.

Das lytische Enzym kann ebenfalls als Zusatzstoff in Waschmitteln eingesetzt werden, um besonders im Bereich von Temperaturen unter 40°C Bakterien abzutöten.

Sofern Bakterien für den Aufschluss biologischen Materials eingesetzt werden, werden Temperaturen von 25 bis 30°C, vorzugsweise 30°C sowie pH-Werte von 5,75 bis 10,0, vorzugsweise 7,7 bevorzugt.

Im Fall der Verwendung des erfindungsgemäßen Enzyms mit der Sequenz aus SEQ ID Nr. 1, 5, 6 und/oder 7 wird ein pH-Wert von 5,0 bis 12,5, vorzugsweise 9,0 bis 10,0, und einem Temperaturbereich von 30 bis 40°C, vorzugsweise 37°C bevorzugt.

Die Erfindung wird anhand der Ausführungsbeispiele und der dazugehörigen Figuren näher erläutert.

Die Figuren zeigen:
- Figur 1: eine Wachstumskurve des erfindungsgemäßen Organismus sowie eine graphische Darstellung dessen autolytischer Aktivität,
- Figur 2 und 3: eine graphische Darstellung der Entwicklung der Zellzahlen bei gemeinsamer Inkubation des erfindungsgemäßen Organismus und M. luteus, E. coli oder S. cerevisiae in Wasser (Figur 2) und in Medium (Figur 3),
- Figur 4: die Ergebnisse einer MonoQ-Chromatographie aus einer Fermentation mit dem erfindungsgemäßen Mikroorganismus im Medium,
- Figur 5: in graphischer Form die Aktivitäten des erfindungsgemäßen Enzyms (aus MonoQ-Chromatographie) gegen M. luteus- und Lysobacter-Zellen,
- Figur 6: die Ergebnisse einer Gel-Filtration (Fraktionen 24 und 25),
- Figur 7: in graphischer Form die Aktivitäten des erfindungsgemäßen Enzyms (aus Gelfiltration) gegen M. luteus- und Lysobacter-Zellen,
- Figur 8: Lysehöfe auf Belebtschlamm des erfindungsgemäßen Mikroorganismus im Gegensatz zu den beiden Vergleichsstämmen L. enzymogenes 495 und L. enzymogenes AL1,
- Figur 9: die Ergebnisse einer Gelfiltration (Fraktionen 21 und 22) und
- Figur 10: in graphischer Form die Aktivitäten des Enzyms mit α-lytischer Aktivität gegen M. luteus- und Lysobacter-Zellen.

Die nachstehenden Sequenzprotokolle sind Bestandteil der vorliegenden Lehre.
SEQ ID Nr. 1: 39 Aminosäuren aus dem N-terminalen Bereich des aufgefundenen Enzyms aus dem erfindungsgemäßen Mikroorganismus
SEQ ID Nr. 2: Die aus der SEQ ID Nr. 1 abgeleitete Nucleinsäuresequenz
SEQ ID Nr. 3: 18 Aminosäuren aus dem N-terminalen Bereich einer α-lytischen Protease aus dem erfindungsgemäßen Mikroorganismus
SEQ ID Nr. 4: Die aus der SEQ ID Nr. 3 abgeleitete Nucleinsäuresequenz
SEQ ID Nr. 5: 5 Aminosäuren des aufgefundenen Enzyms aus dem erfindungsgemäßen Mikroorganismus
SEQ ID Nr. 6: 7 Aminosäuren des aufgefundenen Enzyms aus dem erfindungsgemäßen Mikroorganismus
SEQ ID Nr. 7: 4 Aminosäuren des aufgefundenen Enzyms aus dem erfindungsgemäßen Mikroorganismus

### Beispiel 1: Isolation des Mikroorganismus

Es wurde ein Screening nach Organismen durchgeführt, die extracelluläre, lytische Enzyme bilden. Das Screening wurde wie folgt durchgeführt:

Das Screening wurde mittels Petrischalen durchgeführt, die jeweils drei voneinander getrennte Felder aufwiesen. In einem ersten Screening-Schritt wurden die drei Felder der Petrischale mit autoklavierten Micrococcus-luteus-Zellen, autoklavierten Saccharomyces-cerevisiae-Zellen und autoklavierten Escherichia-coli-Zellen als Futter belegt. Die zu untersuchenden Mikroorganismenstämme wurden jeweils auf jedes der wie vorstehend beschriebenen Felder angeimpft und wachsen gelassen. Für die im folgenden beschriebenen nächsten Screening-Schritte wurden jeweils nur die Stämme verwendet, die gute lytische Aktivität mit dem angegebenen Futter des vorherigen Screeningschrittes aufwiesen.

Im zweiten Screening-Schritt wurden Petrischalen mit lebenden Micrococcus-luteus-Zellen, lebenden Saccharomyces-cerevisiae-Zellen und lebenden Escherichia-coli-Zellen verwendet. In einem dritten Screening-Schritt wurde nicht-autoklavierter Klärschlamm (Ablauf 2. Faulstufe), lebende Saccharomyces-cerevisiae-Zellen und Magermilch verwendet, während in dem vierten Screening-Schritt autoklavierter Klärschlamm (Ablauf 2. Faulstufe), nicht-autoklavierter Klärschlamm (Ablauf 1. Faulstufe) und autoklavierter Klärschlamm (Ablauf 1. Faulstufe) verwendet wurden. Nach Durchlaufen dieser vier Screening-Schritte wurden 8 Isolate mit hoher lytischer Aktivität isoliert, wobei das Isolat mit der höchsten lytischen Aktivität weiter verwendet wurde.

Es konnte ein besonders aktiver Stamm mit der Bezeichnung Stamm 752 isoliert werden, der aus einer Schafweide im Schwarzwald stammt. Dieses Isolat zeigt gegen die beiden getesteten Gram-positiven Bakterien Micrococcus luteus und Bacillus subtilis sehr gute, gegen die Hefe Saccharomyces cerevisae gute und auch gegen die Gram-negativen Bakterien Escherichia coli und Pseudomonas acidovorans lytische Aktivität. Der Organismus erschien in zwei unterschiedlichen Kolonieformen, nämlich einer weißen Kolonie mit schleimigem Wachstum auf Magermilchagar, wobei sich Lysehöfe bilden und einer gelblichen Kolonie, die ein ebenfalls gleitendes Wachstum zeigte, wobei dieses Wachstum jedoch nicht schleimig war. Eine Lysehofbildung auf Futterbakterien konnte in letzterem Fall nicht beobachtet werden. Morphologische und stoffwechselphysiologische Untersuchungen zeigten, dass es sich um ein Gram-negatives, Oxidase-positives stäbchenförmiges Bakterium handelt, welches strikt aerob lebt. Cellobiose, Chitin, Glucose, Maltose, Malat und β-Hydroxybutyrat können verwertet werden, nicht jedoch Mannose, Xylose, Carboxymethylcellulose, Cellulose, Arabinose oder L-Histidin. Es konnte ferner die Fähigkeit zur Hydrolyse von Gelatine, DNA, Tween® 80 und Esculin beobachtet werden. Der Organismus ist ferner Catalase-positiv, Urease- und ADH-negativ. Durch 3%ige KOH wird er lysiert. Bei Natriumchlorid-Konzentrationen von 7% oder darüber kann der isolierte Stamm nicht wachsen. Der OF-Test ergab einen oxidativ-positiven und fermentativnegativen Stoffwechsel, wobei der Voges-Proskauer-Test zum Glucosestoffwechsel negativ verlief. Der Organismus bildete weder Sulfid noch Indol. Es konnte eine sehr starke Hydrolyse von Casein beobachtet werden, ebenso wie der Citrattest (Agartest) und die Hämolyse von Schafsblut positiv verlief.

Die morphologischen Untersuchungen zeigten, dass es sich um ein stäbchenförmiges Bakterium mit einer Breite von 0,5 bis 0,7 µm und einer Länge von 1,5 bis 4,0 µm handelt. Es konnten gleitende Ausläufer am Kolonierand beobachtet werden, wobei ältere Kolonien weder Furchen zeigten noch gelappt erschienen. Nach zwei Wochen Inkubation setzt Autolyse auf der Mediumplatte ein; eine Gleitbewegung in Richtung nährstoffreicheren Mediums konnte beobachtet werden. Der isolierte Organismus kommt in Form weißer und gelber Kolonien vor, es gibt keinerlei Hinweise auf Begeißelung oder Sporenbildung. Eine partielle Sequenzierung der 16S rDNA zeigt eine hundertprozentige Übereinstimmung zu der von Lysobacter enzymogenes. Es handelt sich daher vermutlich um eine Unterart von Lysobacter enzymogenes, die jedoch insbesondere makroskopische Unterschiede zu den Arten Lysobacter enzymogenes 495 (ATCC 29487) und Lysobacter enzymogenes AL1 (ATCC 27796) aufweist oder einen dieser Art funktionell oder morphologisch ähnlichen Mikroorganismus.

Der isolierte Mikroorganismus wurde bei der DSMZ am 22. Juni 1999 in Braunschweig, Deutschland, unter der Nr. DSM 12887 hinterlegt.

### Beispiel 2: Fermentationsbedingungen

Der Organismus wird auf PC-Medium (5,0 g Caseinpepton, 2,5 g Hefeextrakt ad 1000 ml A. dest., pH 7,7) wachsen gelassen. Durch Zugabe von 5,0 g Glucose kann die maximal erreichte Lebendzellzahl erhöht werden, wobei die Enzymbildung um etwa 15 % steigt. Das gebildete Enzym ist autolytisch und wird durch den Organismus selbst induziert.

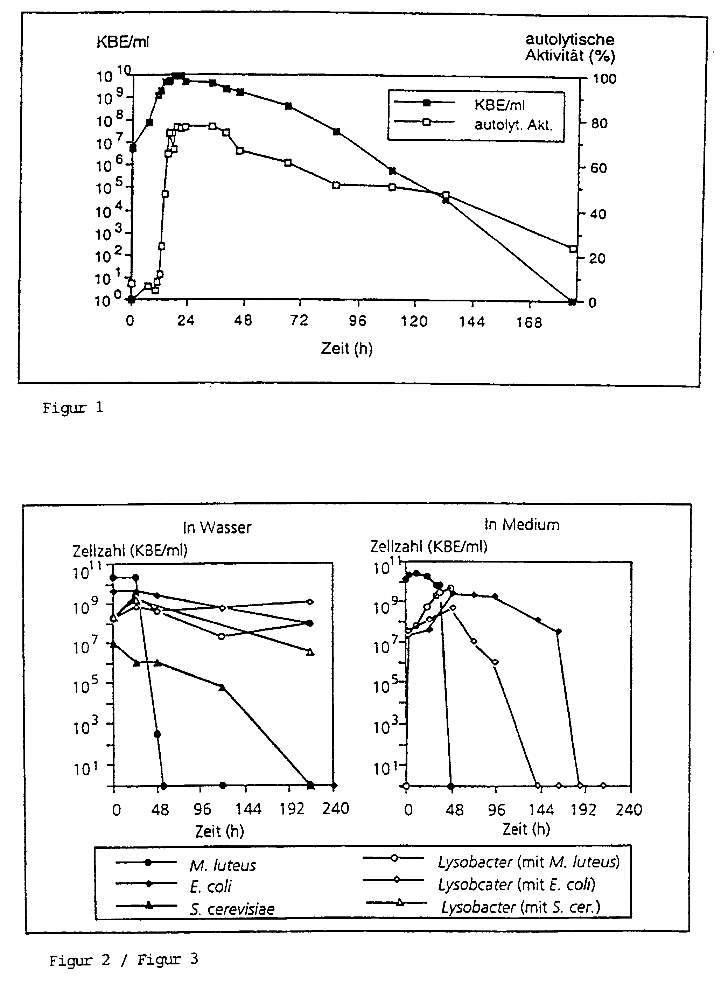

Das Isolat des Mikroorganismus geht nach einer lag-Phase von zirka 8 Stunden in eine 9-stündige Wachstumsphase mit Wachstumsraten von 0,15 h⁻¹ über. Nach einer stationären Phase von maximal 30 Minuten beginnt die Autolysephase, nach weiteren 7 Tagen Fermentationsdauer sind im Reaktor keine lebenden Zellen mehr nachweisbar (Figur 1). Zu Beginn der autolytischen Phase steigt der pH-Wert auf Werte über pH 8 an. Bei externer Steuerung des pH-Wertes auf Werte unter pH 8, ist keine Lyse festzustellen, die Zellzahl bleibt ebenso wie die Enzymaktivität über Tage hinweg konstant. Durch die Zugabe von Phosphat kann die Autolyse des Organismus beschleunigt werden.

Der isolierte Stamm zeigt bei einem pH-Wert von 7,7 optimales Wachstum, wobei der isolierte Stamm befähigt ist, den pH-Wert zu Beginn des Wachstums auf pH 7,7 abzusenken beziehungsweise anzuheben, wenn der vorliegende pH-Wert vom Optimum abweicht. Die pH-Toleranz liegt daher zwischen Werten von pH 5,75 und pH 10,0.

Die günstigste Wachstumstemperatur für den isolierten Stamm liegt bei Temperaturen von 25 bis 30°C mit einem Optimum bei 30°C, wobei bei dem isolierten Stamm aufgefunden werden konnte, dass schon bei 33°C das Wachstum stark eingeschränkt ist und ab 40°C kein Wachstum mehr stattfindet. Der aufgefundene Organismus ist daher aufgrund dieser Temperatur-Empfindlichkeit für den Menschen unbedenklich.

Zum Erreichen einer maximalen Enzymausbeute ist eine Fermentation im Propellerschlaufenreaktor besonders vorteilhaft. Selbstverständlich ist es auch möglich, Enzyme im Rührkesselreaktor zu bilden. Maximale autolytische Aktivität wurde bei einem Leistungseintrag von 1,1 kW/m³ mit einer Begasungsrate von 2 l Luft/min im Propellerschlaufenreaktor ermittelt. Ein geringerer Leistungseintrag kann durch höheren Lufteintrag, der seinerseits sowohl zur besseren Sauerstoffversorgung als auch zur besseren Durchmischung beiträgt, ausgeglichen werden.

### Beispiel 3: Untersuchungen zur lytischen Aktivität des isolierten Bakterienstammes

Es wurden folgende Untersuchungen zur lytischen Aktivität mittels des isolierten Bakteriums durchgeführt

Die Untersuchungen wurden mit frischen und mit thermisch inaktivierten Futterorganismen sowohl in Wasser als auch in PC-Medium bei 30°C im Schikane-Schüttelkolben durchgeführt.

Die Aktivität des Enzyms wurde photometrisch als prozentuale Abnahme der durch die lysierten Bakterien verursachten Trübung bei 500 nm bestimmt.

Die Untersuchungen zeigten, dass thermisch inaktivierte Gram-positive Bakterien mit einer Zelldichte von 10¹⁰ KBE/ml (koloniebildende Einheiten) in 24 Stunden vollständig lysiert werden konnten. Lebende B. subtilis-Zellen derselben Dichte wurden in etwa 30 Stunden aufgelöst, während lebende M. luteus-Zellen in 60 Stunden aufgelöst wurden. Die Lysegeschwindigkeit konnte auf wenige Stunden verkürzt werden, wenn der erfindungsgemäße Stamm konditioniert war, das heißt vor Untersuchungsbeginn bereits lytisch aktiv war. Wenn also nach einer bereits erfolgten Lyse weitere frische, nicht vorbehandelte zu lysierende Bakterien zugegeben wurden, konnten erheblich verringerte Lysezeiten festgestellt werden. Dies kann daran liegen, dass aufgrund der Konditionierung die notwendigen lytischen Enzyme bereits gebildet worden waren und ihre Wirkung sofort einsetzen konnte.

Für die Lyse thermisch inaktivierter Gram-positiver Bakterien machte es keinen Unterschied, ob die zu lysierenden Bakterien in bidestilliertem Wasser, in Leitungswasser oder Vollmedium aufgenommen worden waren. Auf die Autolyse des erfindungsgemäßen Stammes selbst hatte das Medium jedoch einen erheblichen Einfluss. Wurden die Bakterien in Trinkwasser aufgenommen, setzte die Autolyse erst nach mehreren Wochen ein, während im Komplettmedium bereits nach 20 Fermentationsstunden Autolyse beobachtet werden konnte (Figuren 2 und 3).

Ohne durch die Theorie beschränkt zu sein, ist es denkbar, dass die Anwesenheit von Fettsäuren für die lytische Wirksamkeit besonders gegen Gram-negative Bakterien von Bedeutung ist. Unverzweigte Fettsäuren zeigen einen stärkeren Einfluss auf die lytische Wirksamkeit als verzweigte. Möglicherweise erhöhen Fettsäuren die Permeabilität der Zellwände der zu lysierenden Bakterien, wodurch der Angriff der Proteasen erleichtert wird. Bei Untersuchungen mit gewaschenen zu lysierenden Bakterien in Wasser ohne Anwesenheit von Fettsäuren konnte festgestellt werden, dass keine Lyse Gram-negativer Bakterien und keine Autolyse stattfinden konnte. Ein Vergleich der Zeitpunkte der Zellabnahme der verschiedenen zu lysierenden Bakterien, wie in den Figuren 2 und 3 ersichtlich, zeigt, dass Gram-positive Bakterien während der Wachstumsphase der erfindungsgemäßen Spezies lysiert werden, wohingegen die Abnahme der E. coli-Zellen während der autolytischen Phase der erfindungsgemäßen Spezies zu beobachten ist. Die Lyse der Hefezellen erfolgt über neun Tage hinweg.

### Beispiel 4: Charakterisierung des gebildeteten Enzyms mit der SEQ ID Nr. 1

Die nachfolgenden Untersuchungen wurden mit aufkonzentriertem Rohextrakt durchgeführt. Zur Gewinnung des Rohextraktes wurde eine Fermentation mit dem neu aufgefundenen Stamm in PC-Medium nach 40 Stunden beendet, da zu diesem Zeitpunkt maximale Enzymkonzentration vorlag (vergleiche Figur 1). Die Zellen wurden in einer Beckman-Zentrifuge (J2-21M/E, Beckman München) bei 15000 g abgetrennt, anschließend wurde der Überstand mittels eines 0,2 µm Hohlfasermoduls (Firma Microdyn, Wuppertal) sterilfiltriert. Der zellfreie Extrakt wurde über eine 5 kD-Ultrafiltrations-Membran (Firma Sartorius, Göttingen) von 16 1 auf 100 ml aufkonzentriert. Der Rohextrakt wurde für die Stabilitäts-Untersuchungen 1/20 mit 50 mM Tris-HCl-Puffer verdünnt.

Die Bestimmung des pH-Wert-Optimums erfolgte in je 20 mM Citratpuffer (pH 4,0 bis 6,5), Tris-HCl (pH 7,0 bis 9,5) und Tris-NaOH (pH 10,0 bis 13,0) über 5 Tage.

Das pH-Wert Optimum liegt bei 9,0 bis 10,0, wobei eine Aktivität über 50% noch bei pH-Werten von 5,0 bis 12,5 und Aktivität über 10% bei einem pH-Wert von 4,5 bis 13,0 beobachtet werden konnte. Der Aktivitätsverlust im sauren Bereich ist reversibel und kann durch Umpuffern kompensiert werden.

Bei dem isolierten Enzym wurde ein TemperaturOptimum von 37°C ermittelt. Bei dieser Temperatur sind die Enzyme auch über mehrere Tage hinweg stabil. Bei 55°C ist die Aktivität bei einstündiger Inkubation geringfügig geringer, nach eintägiger Inkubation beträgt sie jedoch nur noch 20% der ursprünglichen Aktivität.

Die Aktivität des Enzyms wurde erst bei einer Natriumchlorid- beziehungsweise KCl-Konzentration von über 100 mM vermindert, bei 1 M-Salzkonzentration hatte sie um die Hälfte abgenommen.

Das Enzym wird durch eine Protease aus B. licheniformis inaktiviert. Phenylmethylsulfonyl-fluorid (PMSF), ein Inhibitor von Serin-Proteasen, hat keinen Einfluss auf die autolytische Aktivität. Unter anaeroben Bedingungen sinkt die lytische Aktivität gegen Gram-negative Bakterien.

### Beispiel 5: Isolierung des lytischen Enzyms mit der SEQ ID Nr. 1

Eine Reindarstellung des lytischen Enzyms wurde wie folgt durchgeführt.

Mit dem in Beispiel 4 aufkonzentrierten Rohextrakt wurde zunächst eine Ionenaustausch-Chromatographie mit einer FPLC (Fast Protein Liquid Chromatographie, Firma Pharmacia, Freiburg) bei 4°C durchgeführt. Dabei wurde eine MonoQ-Säule HR10/10 eingesetzt, die einen starken Anionenaustauscher darstellt, eine Partikelgröße von 10 µm und bindende Gruppen -CH₂-N-(CH₃)₃ aufweist. Als Laufmittel A wurde 50 mM Tris-HCl (pH 9,0) verwendet. Laufmittel B war 50 mM Tris-HCl mit 1,0 M NaCl (pH 9,0). Die Fraktionsgrößen betrugen 1 ml. Das Trennprogramm ist der nachstehenden Tabelle zu entnehmen.

| Trennprogramme für die Ionenaustauschchromatographie | | |
|---|---|---|
| min | Konzentration Laufmittel B | Fließgeschwindigkeit |
| 0 - 2 | 0 % | 1,0 ml/min |
| 2 - 14 | 0 - 30 % | 1,0 ml/min |
| 14 - 24 | 30 - 100 % | 1,0 ml/min |
| 24 - 27 | 100 % | 1,0 ml/min |
| 27 - 30 | 100 - 0 % | 1,0 ml/min |
| 30 - 35 | 0 % | 1,0 ml/min |

Auf die Säule wurden 2 ml des 1/3 in Tris-HCl-Puffer verdünnten Rohextrakts aufgetragen, der, wie erwähnt, durch Aufkonzentrierung mittels Ultrafiltration aus einer Fermentation des erfindungsgemäßen Mikroorganismus in Vollmedium gewonnen worden war.

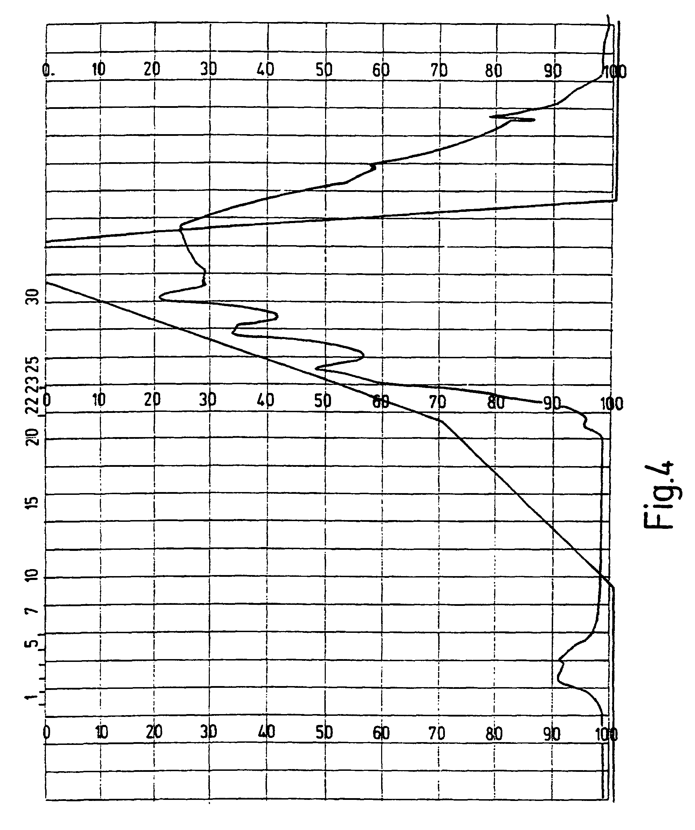

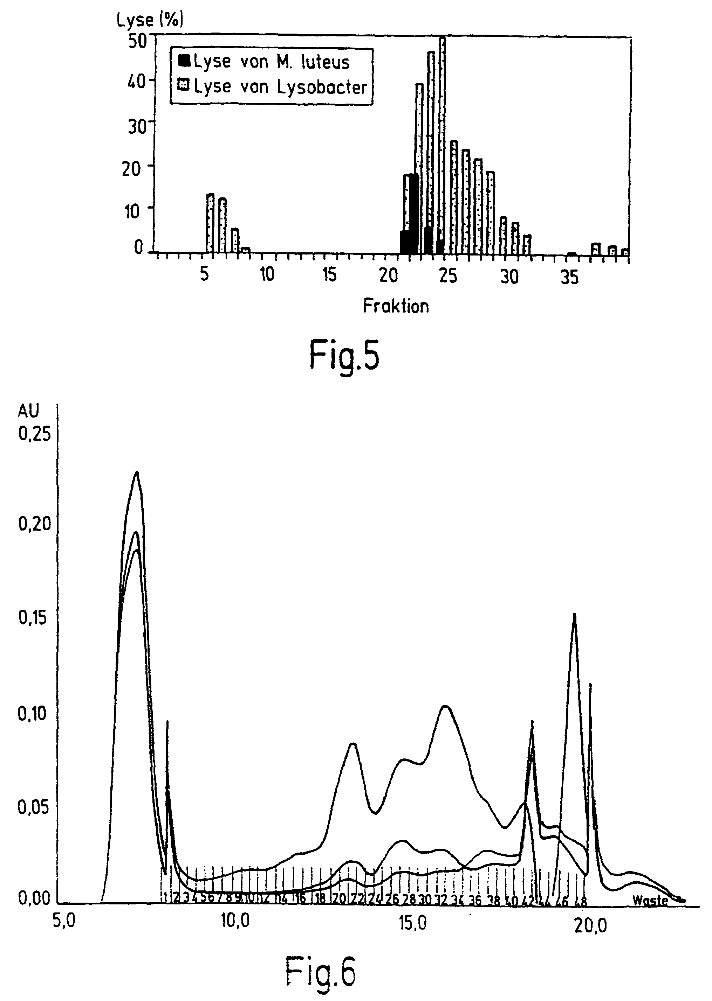

Die Figur 4 zeigt eine typische MonoQ-Chromatographie einer Probe aus einer Fermentation mit dem aufgefundenen Stamm in PC-Medium, das heißt einer Probe aufkonzentrierten Rohextrakts nach Beispiel 4. Die Figur 5 zeigt die Enzymaktivitäten der verschiedenen erhaltenen Fraktionen gegen M. luteus und Lysobacter. Die Fraktionen 24 und 25, die eine hohe autolytische Aktivität, also Aktivität gegen Gram-negative Bakterien, und wenig Aktivität gegen Gram-positive Bakterien zeigten (Figur 5), wurden für eine Gel-Chromatographie vereinigt.

Für die Gelfiltration wurde eine in das Smart-System (HPLC, LKB µ Separation Unit, Firma Pharmacia) eingebaute Superose 12-Säule HR 10/30 (Firma Pharmacia, Freiburg) eingesetzt. Das Säulenmaterial war Dextran mit 12 % quervernetzter Agarose und Partikelgrößen von 13 bis 15 µm. Die Filtration wurde bei 4°C durchgeführt, wobei das Probeauftragsvolumen 1 ml, das Fraktionsvolumen 250 µl und die Durchflussgeschwindigkeit 0,4 ml/min betrug. Die Extinktion wurde bei 214 nm, 256 nm und 280 nm gemessen. Als Laufmittel wurde 150 mM Di-Ammoniumhydrogencarbonat-Puffer mit einem pH-Wert von 8,5 verwendet. Als Standards wurden Ferritin, Catalase, BSA, Cytochrom C und Vitamin B₁₂ verwendet.

Die Figur 6 zeigt das Ergebnis einer Gelfiltration mit einer Superose 12-Säule der Fraktionen 24 und 25 der vorgenannten Ionenaustausch-Chromatographie (vergleiche Figur 4).

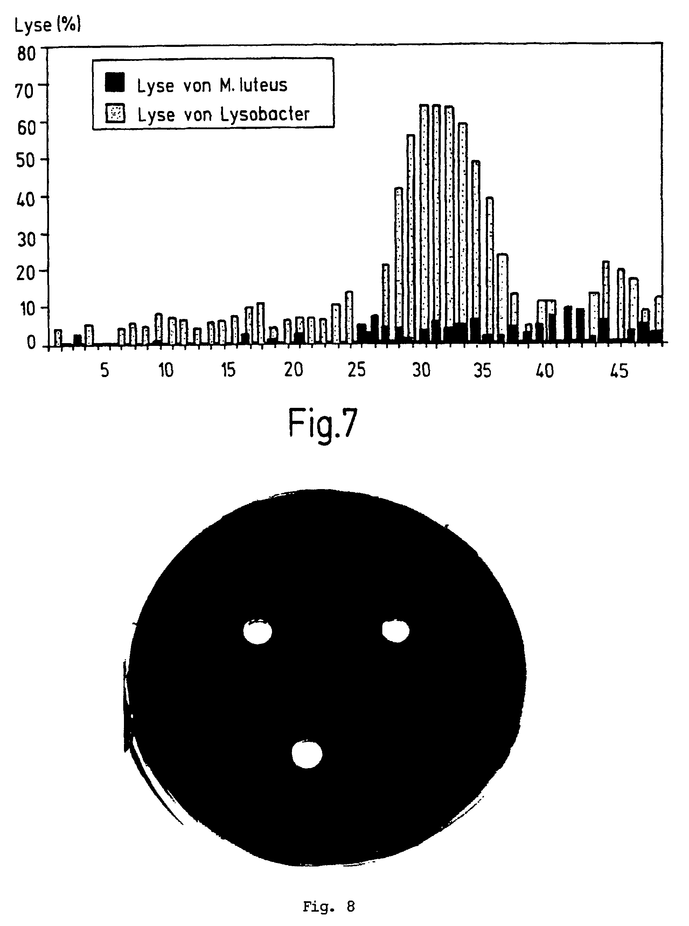

Die Figur 7 zeigt die Enzymaktivitäten der erhaltenen Fraktionen gegen M. luteus und Lysobacter-Zellen in den einzelnen Fraktionen aus der in Figur 6 dargestellten Gelfiltrationen (eingesetzte Probe: Fraktionen 24 und 25 der Ionenaustausch-Chromatographie).

In den Fraktionen 28 bis 32 war eine deutlich höhere Aktivität nachzuweisen als in den restlichen Fraktionen, die maximal 12% Lyse-Aktivität zeigten. In den Fraktionen 29, 30 und 31 betrug die autolytische Aktivität jeweils 65%. Diese Probevolumina entsprechen einer Molekülgröße von etwa 15000 bis 20000 Da. Sie wurden daher vereinigt und die Enzyme auf eine PVDF-Membran geblottet (ProBlot, Firma PE Biosystems).

### Beispiel 6: Aminosäure-Sequenzierung

Zur Ermittlung der Proteinsequenz wurde der Sequenzator 473A (Firma Applied Biosystems) eingesetzt, der die Aminosäuresequenz mittels Edman-Abbau ermittelt. Die Cyklen und Chemikalien wurden nach Herstellerangaben eingesetzt.

Das lytische Enzym lag in hoher Konzentration vor, so dass mittels Edman-Abbaus die N-terminalen Aminosäuresequenzen sequenziert werden konnten. Dabei wurde mittels MonoQ und Superose 12 aufgereinigten Enzyms vorgegangen. Die ermittelte Sequenz von 39 Aminosäuren ist in SEQ ID Nr. 1 dargestellt. Die nicht ermittelte Aminosäure an Position 11 ist vermutlich Cys, da der Sequenzator Cys nicht erkennt. Weitere Aminosäuresequenzen dieses Enzyms sind in den SEQ ID Nr. 5, 6 und 7 dargestellt. Diese Aminosäurensequenzen stammen aus dem internen Bereich des Enzyms. An Position 2 der SEQ ID Nr. 5 steht vermutlich ein F (Phe) oder D (Asp) und an Position 3 der SEQ ID Nr. 6 ein E (Glu) oder C (Cys).

Die ermittelte Sequenz wurde zu bisher bekannten Protein-Sequenzen verglichen, wobei keine Homologie zu bisher bekannten Proteinen von zum Beispiel Lysobacter gefunden wurde. Mittels der ermittelten Aminosäuresequenz der SEQ ID Nr. 1 sowie der davon abgeleiteten Nucleotidsequenz (SEQ ID Nr. 2) ist der Einsatz einer Nucleotidsonde möglich, mit Hilfe derer das vollständige Gen für das aufgefundene Protein cloniert und für die gentechnische Herstellung des Proteins eingesetzt werden kann.

Die Aufreinigung und Identifizierung des neuen Proteins wurde im Abstand von mehreren Monaten aus unterschiedlichen Fermentationen wiederholt und bestätigt. Die größte Übereinstimmung in der Aminosäure-Sequenz konnte mit 39% zu einer Peptidyl-Lys-Metalloendopeptidase aus Pleurotus ostreatus bei einem Fragment aus 31 Aminosäuren festgestellt werden. Enzyme der Metalloendopeptidase-Familie zeichnen sich durch eine hohe Temperatur-Stabilität, eine Resistenz gegen denaturierende Agentien sowie eine Aktivität über einen großen pH-Bereich aus, was mit den Angaben zu dem vorliegenden Enzym übereinstimmt.

### Beispiel 7: Bestimmung des Molekulargewichtes

Die Bestimmung des Molekulargewichtes mittels MALDI TOF erfolgte mit einer Sinapinsäure-Matrix unter Vakuum. Es wurden 2 µl Sinapinsäure zu 1 µl Proteinlösung gegeben.

Die Parameter waren wie folgt:

| | | |
|---|---|---|
| Masse-Bereich | 100.000 Da Ion Optics | 28.0/7.0 kV |
| Masse-Filter | 5.000 Da Detektor | -4,75 kV |
| Daten-Intervall | 4.0 nsec Digitizer | 1.000 mVFS |
| Polarität | positiv Filter | keiner |

Das aufgereinigte und sequenzierte lytische Enzym der vorliegenden Lysobacter enzymogenes Art weist laut MALDI TOF-Bestimmung ein Molekulargewicht von 18.538 Da auf.

### Beispiel 8: Anwendung für Zellaufschluss

Das erfindungsgemäß gebildete Enzym weist sehr hohe lytische Aktivität gegen Gram-negative und gute lytische Aktivität gegen Gram-positive Bakterien auf. Das Enzym kann zum Aufschluss von Zellen verwendet werden, beispielsweise auch zur enzymatischen Vorbehandlung von Klärschlamm, um die anaerobe Umsetzung beispielsweise in einem Faulturm zu erhöhen. Untersuchungen mit Belebtschlamm zeigten, dass die Belebtschlammorganismen, wie in Figur 8 gezeigt, lysiert werden.

Figur 8 zeigt im Detail die Lysehöfe auf Belebtschlamm des erfindungsgemäß isolierten Stammes (unten mittig) (DSM 12887) im Gegensatz zu den beiden Vergleichsstämmen L. enzymogenes 495 (oben links) (DSM 2043) und L. enzymogenes AL1 (oben rechts) (DSM 1895).

### Beispiel 9: Identifizierung, Isolierung und Aminosäuresequenzierung einer α-lytischen Protease (SEQ ID Nr. 3) aus dem erfindungsgemäßen Stamm

Mittels des in Beispiel 4 durch Aufkonzentrierung durch Ultrafiltration aus einer Fermentation des neu aufgefundenen Stammes in Vollmedium gewonnenen verdünnten Rohextraktes wurde eine Ionenaustausch-Chromatographie wie in Beispiel 5 beschrieben durchgeführt. Die Ionenaustausch-Chromatographie wurde mit 2 ml des 1/3 in Puffer verdünnten Rohextrakts durchgeführt, welches aus einer nach 40 Stunden beendeten Fermentation des erfindungsgemäßen Stammes in Vollmedium ohne Zugabe von Futterbakterien stammte, so dass das lytische Enzym in maximaler Konzentration vorlag.

Die Figur 4 zeigt eine typische MonoQ-Chromatographie einer Probe aus einer Fermentation mit Lysobacter in PC-Medium, wobei in Figur 5 die Enzymaktivitäten gegen M. luteus und Lysobacter-Zellen in den Fraktionen der in Figur 4 dargestellten MonoQ-Chromatographie dargestellt ist.

Durch Ionenaustausch-Chromatographie konnte eine gute Aufreinigung erzielt werden. Die Fraktionen 21 und 22 mit vergleichsweise hoher lytischer Aktivität gegen Gram-positive Bakterien aus Figur 4 wurden vereinigt und über eine Superose 12-Säule aufgereinigt. Die lytische Aktivität gegen L. enzymogenes war in allen Fraktionen höher als gegen M. luteus.

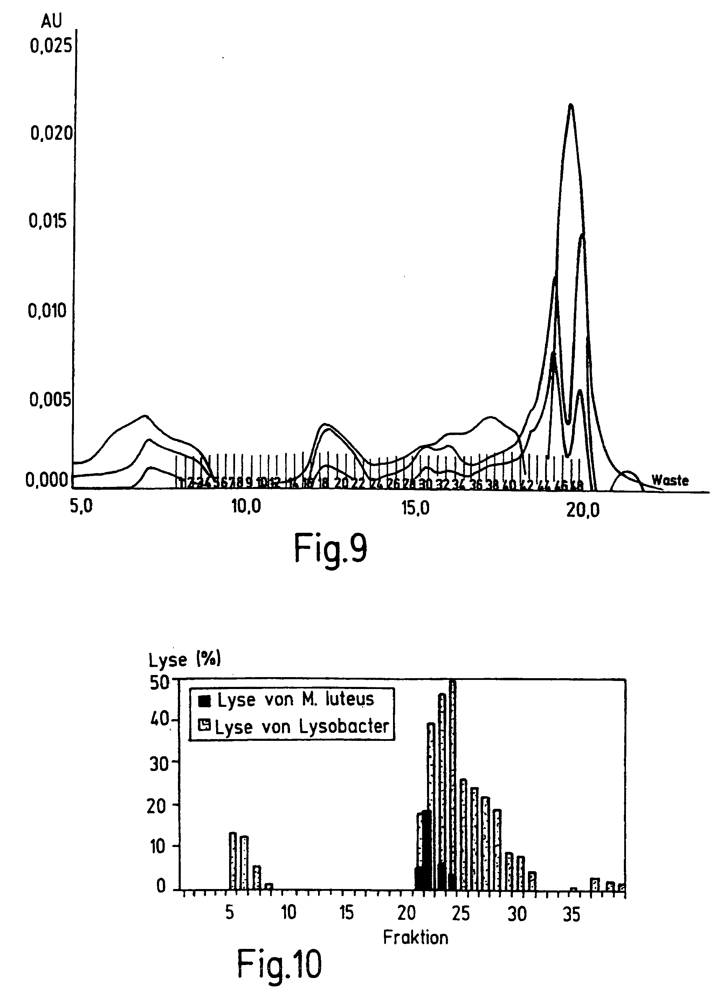

Die Figur 9 zeigt das Ergebnis einer Gelfiltration mit einer Superose 12-Säule mit den Fraktionen 21 und 22 der vorgenannten Ionenaustausch-Chromatographie.

Die Figur 10 zeigt die Enzymaktivitäten gegen M. luteus und Lysobacter-Zellen in den Fraktionen der in Figur 9 dargestellten Gelfiltration.

Die Sequenz der Aminosäuren wurde mittels eines automatischen Sequenzators durch Edman-Abbau ermittelt, wobei die N-terminalen 18 Aminosäuren identifiziert werden konnten. Die Probe wurde vor der Sequenzierung nicht alkyliert, wodurch ein Cystein nicht erkannt werden kann. Die nicht identifizierte Aminosäurenposition 17 könnte demnach Cystein sein. Es liegt eine hohe Sequenzhomologie von 83% zu der α-lytischen Protease von Lysobacter enzymogenes vor (Epstein und Wensink, J. of Biol. Chem., 263 (32) (1988), 16586-16590). Die Aminosäuresequenz des vorliegenden α-lytischen Enzyms wird in SEQ ID Nr. 3 gezeigt und die davon abgeleitete Nucleinsäuresequenz in SEQ ID Nr. 4. Letztere kann zur Isolierung des vollständigen Gens verwendet werden.

## Patentansprüche

1. Protein, das die Aktivität eines lytischen Enzyms aufweist und in der Lage ist, die Zellwand oder diese aufbauende Substanzen zu spalten, ausgewählt aus der Gruppe bestehend aus
a) einem Protein aus Lysobacter enzymogenes, das die in SEQ ID Nr. 1, 5, 6 und 7 dargestellten Aminosäuresequenzen umfasst,
b) einem Protein mit einer Aminosäuresequenz, die zu mindestens 90% identisch ist zu den in SEQ ID Nr. 1, 5, 6 und 7 dargestellten Aminosäuresequenzen, und
c) einem Derivat des unter a) und b) genannten Proteins, das sich durch chemische Modifikationen einer oder mehrerer Aminosäuren auszeichnet.

2. Protein nach Anspruch 1, wobei das Protein ein mittels HPLC-Chromatographie ermitteltes Molekulargewicht von 15.000 bis 20.000 Da, ein Temperaturoptimum von 37°C und ein pH-Optimum von 9,0 bis 10,0 aufweist.

3. Gemisch aus mindestens zwei Proteinen, die jeweils die Aktivität lytischer Enzyme aufweisen und in der Lage sind, die Zellwand oder diese aufbauende Substanzen zu spalten, wobei ein Protein eines der Ansprüche 1 oder 2 und zumindest ein weiteres Protein ausgewählt ist aus der Gruppe, bestehend aus der β-lytischen Metallo-Endopeptidase von Lysobacter enzymogenes und einer α-lytischen Protease von Lysobacter enzymogenes mit einer Aminosäuresequenz, dargestellt in SEQ ID Nr. 3 oder einer mutanten Variante oder eines Derivats davon.

4. Bakterium mit der Fähigkeit zur Bildung und/oder Ausscheidung eines lytischen Enzyms nach einem der Ansprüche 1 oder 2, insbesondere hinterlegt bei der DSMZ unter der Nr. DSM 12887.

5. Nucleinsäuremolekül, das ein lytisches Enzym nach einem der Ansprüche 1 oder 2 codiert, umfassend eine Nucleinsäuresequenz, ausgewählt aus der Gruppe bestehend aus
a) einer Nucleinsäuresequenz, die eine Aminosäuresequenz nach SEQ ID Nr. 1, 5, 6 und/oder 7 codiert oder deren komplementäre Sequenz,
b) einer Nucleinsäuresequenz nach SEQ ID Nr. 2 oder deren komplementäre Sequenz,
c) einer Nucleinsäuresequenz, die mit einer der Sequenzen nach a) oder b).hybridisiert oder deren komplementäre Sequenz und
d) einer Nucleinsäuresequenz, die aufgrund der Degeneration des genetischen Codes von der Sequenz unter a), b) oder c) abweicht.

6. Vektor, enthaltend ein Nucleinsäuremolekül nach Anspruch 5.

7. Vektor nach Anspruch 6, wobei das Nucleinsäuremolekül mit regulatorischen Elementen operativ verknüpft ist, die die Transcription und Synthese einer translatierbaren RNA in pro- und/oder eucaryotischen Zellen gewährleisten.

8. Wirtszelle, enthaltend einen Vektor nach einem der Ansprüche 6 oder 7.

9. Verfahren zur Herstellung eines Proteins, das die Aktivität eines lytischen Enzyms aufweist und in der Lage ist, die Zellwand oder diese aufbauende Substanzen zu spalten, insbesondere eines Proteins nach einem der Ansprüche 1 oder 2, wobei eine Wirtszelle nach Anspruch 8 unter Bedingungen kultiviert wird, die die Synthese des Proteins erlauben und das Protein aus den kultivierten Zellen und/oder dem Kulturmedium isoliert wird.

10. Verfahren zur Herstellung eines Proteins, das die Aktivität eines lytischen Enzyms aufweist und in der Lage ist, die Zellwand oder diese aufbauende Substanzen zu spalten, insbesondere eines Proteins nach einem der Ansprüche 1 oder 2, wobei ein Bakterium nach Anspruch 4 unter Bedingungen kultiviert wird, die die Synthese das Proteins erlauben und das Protein aus den kultivierten Zellen und/oder dem Kulturmedium isoliert wird.

11. Protein, hergestellt nach einem der Verfahren der Ansprüche 9 oder 10.

12. Antikörper, der spezifisch ein Protein nach einem der Ansprüche 1, 2 oder 11 erkannt und bindet.

13. Verfahren zum Aufschluss von biologischen Material, insbesondere Zellen oder Macromolekülen, wobei ein Bakterium nach Anspruch 4, ein Protein nach einem der Ansprüche 1, 2 oder 11 oder/und ein Proteingemisch nach Anspruch 3 dem aufzuschließenden biologischen Material hinzugegeben und unter Bedingungen inkubiert wird, die den Aufschluss des biologischen Materials erlauben.

14. Verwendung eines Proteins nach einem der Ansprüche 1, 2 oder 11 zur enzymatischen Vorbehandlung von Klärschlamm.

## Claims

1. Protein, which has the activity of a lytic enzyme and is capable of splitting the cell wall or substances forming this, selected from the group comprising
a) a protein from lysobacter enzymogenes, which comprises the amino acid sequences represented in SEQ ID No. 1, 5, 6 and 7,
b) a protein with an amino acid sequence, which is at least 90% identical to the amino acid sequences represented in SEQ ID No. 1, 5, 6 and 7, and
c) a derivative of the protein given in a) and b), which is distinguished by chemical modifications of one or more amino acids.

2. Protein according to Claim 1, wherein the protein has a molecular weight of 15 000 to 20 000 Da determined by HPLC chromatography, an optimum temperature of 37°C and an optimum pH of 9.0 to 10.0.

3. Mixture comprising at least two proteins, which respectively have the activity of lytic enzymes and are capable of splitting the cell wall or substances forming this, wherein a protein of one of Claims 1 or 2 and at least a further protein is selected from the group comprising the β-lytic metallo-endopeptidase of lysobacter enzymogenes and an α-lytic protease of lysobacter enzymogenes with an amino acid sequence represented in SEQ ID No. 3 or of a mutant variant or derivative thereof.

4. Bacterium with the ability to form and/or excrete a lytic enzyme according to one of Claims 1 or 2, in particular recorded at the DSMZ [German Collection of Microorganisms and Cell Cultures] under No. DSM 12887.

5. Nucleic acid molecule, which codes a lytic enzyme according to one of Claims 1 or 2, comprising a nucleic acid sequence, selected from the group comprising
a) a nucleic acid sequence, which codes an amino acid sequence according to SEQ ID No. 1, 5, 6 and/or 7 or its complementary sequence,
b) a nucleic acid sequence according to SEQ ID No. 2 or its complementary sequence,
c) a nucleic acid sequence, which hybridises with one of the sequences according to a) or b) or its complementary sequence, and
d) a nucleic acid sequence, which deviates from the sequence under a), b) or c) as a result of degeneration of the genetic code.

6. Vector containing a nucleic acid molecule according to Claim 5.

7. Vector according to Claim 6, wherein the nucleic acid molecule is operatively bonded with regulator elements, which assure the transcription and synthesis of a translatable RNA in prokaryotic and/or eukaryotic cells.

8. Host cell containing a vector according to one of Claims 6 or 7.

9. Process for the production of a protein, which has the activity of a lytic enzyme and is capable of splitting the cell wall or substances forming this, in particular a protein according to one of Claims 1 or 2, wherein a host cell according to Claim 8 is cultivated under conditions, which allow the synthesis of the protein, and the protein is isolated from the cultivated cells and/or the culture medium.

10. Process for the production of a protein, which has the activity of a lytic enzyme and is capable of splitting the cell wall or substances forming this, in particular a protein according to one of Claims 1 or 2, wherein a bacterium according to Claim 4 is cultivated under conditions, which allow the synthesis of the protein, and the protein is isolated from the cultivated cells and/or the culture medium.

11. Protein produced according to one of the processes of Claims 9 or 10.

12. Antibody, which specifically recognises and binds a protein according to one of Claims 1, 2 or 11.

13. Process for the breakdown of biological material, in particular cells or macromolecules, wherein a bacterium according to Claim 4, a protein according to one of Claims 1, 2 or 11 and/or a protein mixture according to Claim 3 are added to the biological material to be broken down and incubated in conditions, which allow the breakdown of the biological material.

14. Use of a protein according to one of Claims 1, 2 or 11 for the enzymatic pretreatment of sedimentation sludge.

## Revendications

1. Protéine qui présente l'activité d'une enzyme lytique et est capable de désintégrer la paroi cellulaire ou des substances la constituant, choisie dans le groupe constitué par
a) une protéine de *Lysobacter enzymogenes*, qui comprend les séquences d'aminoacides représentées dans SEQ ID n° 1, 5, 6 et 7,
b) une protéine ayant une séquence d'aminoacides qui est identique à raison d'au moins 90 % aux séquences représentées dans SEQ ID n° 1, 5, 6 et 7, et
c) un dérivé de la protéine mentionnée en a) et b), qui se distingue par des modifications chimiques d'un ou plusieurs aminoacides.

2. Protéine selon la revendication 1,
**caractérisée par**
une masse moléculaire, déterminée par HPLC, de 15 000 à 20 000 Da, un optimum de température de 37°C et un optimum de pH de 9,0 à 10,0.

3. Mélange d'au moins deux protéines qui présentent chacune l'activité d'enzymes lytiques et sont capables de désintégrer la paroi cellulaire ou des substances la constituant, dans lequel une protéine est une protéine de la revendication 1 ou 2, et au moins une autre protéine est choisie dans le groupe constitué par la métallo-endopeptidase β-lytique de *Lysobacter enzymogenes* et une protéase α-lytique de *Lysobacter enzymogenes* ayant une séquence d'aminoacides représentée dans SED ID n° 3, ou un variant mutant ou un dérivé de celle-ci.

4. Bactérie ayant l'aptitude à la formation et/ou à l'excrétion d'une enzyme lytique selon la revendication 1 ou 2, en particulier déposée à la DSMZ (Deutsche Sammlung für Mikroorganismen und Zellkulturen) sous le n° DSM 12887.

5. Molécule d'acide nucléique qui code pour une enzyme lytique selon la revendication 1 ou 2, comprenant une séquence d'acide nucléique choisie dans le groupe constitué par
a) une séquence d'acide nucléique qui code une séquence d'amino-acides selon SED ID n° 1, 5, 6 et/ou 7 ou une séquence complémentaire de celle-ci,
b) une séquence d'acide nucléique selon SEQ ID n° 2 ou une séquence complémentaire de celle-ci,
c) une séquence d'acide nucléique qui s'hybride avec l'une des séquences selon a) ou b) ou une séquence complémentaire de celle-ci et
d) une séquence d'acide nucléique qui, en raison de la dégénérescence du code génétique, s'écarte de la séquence en a), b) ou c).

6. Vecteur contenant une molécule d'acide nucléique selon la revendication 5.

7. Vecteur selon la revendication 6,
**caractérisée en ce que**
la molécule d'acide nucléique est fonctionnellement liée à des éléments régulateurs qui assurent la transcription et la synthèse d'un ARN traduisible dans des cellules procaryotes et/ou des cellules eucaryotes.

8. Cellule hôte contenant un vecteur selon la revendication 6 ou 7.

9. Procédé pour la production d'une protéine qui présente l'activité d'une enzyme lytique et est capable de désintégrer la paroi cellulaire ou des substances la constituant, en particulier d'une protéine selon la revendication 1 ou 2,
selon lequel on cultive une cellule hôte selon la revendication 8 dans des conditions qui permettent la synthèse de la protéine et on isole la protéine à partir des cellules cultivées et/ou du milieu de culture.

10. Procédé pour la production d'une protéine qui présente l'activité d'une enzyme lytique et est capable de désintégrer la paroi cellulaire ou des substances la constituant, en particulier d'une protéine selon la revendication 1 ou 2,
selon lequel on cultive une bactérie selon la revendication 4 dans des conditions qui permettent la synthèse de la protéine et on isole la protéine à partir des cellules cultivées et/ou du milieu de culture.

11. Protéine produite selon l'un des procédés des revendications 9 et 10.

12. Anticorps qui reconnaît spécifiquement et se lie spécifiquement à une protéine selon l'une quelconque des revendications 1, 2 et 11.

13. Procédé pour la désintégration de matière biologique, en particulier de cellules ou de macromolécules,
selon lequel on ajoute à la matière biologique à désintégrer une bactérie selon la revendication 4, une protéine selon l'une quelconque des revendications 1, 2 et 11 et/ou un mélange de protéines selon la revendication 3, et on les met à incuber dans des conditions qui permettent la désintégration de la matière biologique.

14. Utilisation d'une protéine selon l'une quelconque des revendications 1, 2 et 11 pour le prétraitement enzymatique de boues d'épuration.
